# EUROPEAN PATENT SPECIFICATION

(11) **EP 1 399 157 B1**
(45) Date of publication and mention of the grant of the patent: **14.09.2005**
(21) Application number: 02745358.8
(22) Date of filing: 04.06.2002
(51) Int. Cl.: A61K 31/415, A61K 31/55, A61K 31/53, A61K 31/195, A61K 31/19, A61K 31/35, A61K 38/13, A61P 25/08, A61P 25/06, A61P 25/18, A61P 25/22, A61P 25/24

(54) **COMBINATION COMPRISING A P-GP INHIBITOR AND AN ANTI-EPILEPTIC DRUG**
KOMBINATION ENTHALTEND EINEN P-GP HEMMER UND EINEN ANTIEPILEPTISCHEN WIRKSTOFF
COMBINAISON COMPRENANT UN INHIBITEUR DE LA P-GLYCOPROTEINE (P-GP) ET UN MEDICAMENT ANTI-EPILEPTIQUE

(30) Priority: 05.06.2001 GB 0113663
(43) Date of publication of application: 24.03.2004
(73) Proprietor: Novartis AG, 4056 Basel (CH); Novartis Pharma GmbH, 1230 Wien (AT); Löscher, Wolfgang, 30625 Hannover (DE); Potschka, Heidrun, 30173 Hannover (DE)
(72) Inventor: LÖSCHER, Wolfgang, 30625 Hannover (DE); POTSCHKA, Heidrun, 30173 Hannover (DE); SCHMUTZ, Markus, CH-4124 Schoenenbuch (CH)
(74) Representative: de Weerd, Petrus G.W.
(86) International application number: PCT/EP2002/006140
(87) International publication number: WO 2002/098418

(56) References cited:
- CHATTOPADHYAY R N ET AL: "Potentiation of antiepileptic activity of phenytoin by calcium channel blockers against maximal electroshock seizure in mice." INDIAN JOURNAL OF PHARMACOLOGY, vol. 30, no. 5, October 1998 (1998-10), pages 326-328, XP001104851 ISSN: 0253-7613
- V WEGERER J ET AL: "A calcium antagonistic effect of the new antiepileptic drug lamotrigine." EUROPEAN NEUROPSYCHOPHARMACOLOGY, vol. 7, no. 2, 1997, pages 77-81, XP002213651 ISSN: 0924-977X
- TISHLER DAVID M ET AL: "MDR1 gene expression in brain of patients with medically intractable epilepsy." EPILEPSIA, vol. 36, no. 1, 1995, pages 1-6, XP001104813 ISSN: 0013-9580
- KOEHLING R ET AL: "Differential involvement of L-type calcium channels in epileptogenesis of rat hippocampal slices during ontogenesis." NEUROBIOLOGY OF DISEASE, vol. 7, no. 4, August 2000 (2000-08), pages 471-482, XP002213652 ISSN: 0969-9961
- WIEMANN MARTIN ET AL: "Simultaneous blockade of intracellular calcium increases and of neuronal epileptiform depolarizations by verapamil." BRAIN RESEARCH, vol. 734, no. 1-2, 1996, pages 49-54, XP002213653 ISSN: 0006-8993
- POTSCHKA H ET AL: "In vivo evidence for P-glycoprotein-mediated transport of phenytoin at the blood-brain barrier of rats." EPILEPSIA. UNITED STATES OCT 2001, vol. 42, no. 10, October 2001 (2001-10), pages 1231-1240, XP002213654 ISSN: 0013-9580
- PATENT ABSTRACTS OF JAPAN vol. 1996, no. 09, 30 September 1996 (1996-09-30) & JP 08 127541 A (CHUGAI PHARMACEUT CO LTD), 21 May 1996 (1996-05-21)

## Description

The invention relates to a combination which comprises a P-glycoprotein (P-gp) inhibitor selected from [3'-desoxy-3'-oxo-MeBmt]¹-Ciclosporin[3'-desoxy-3'-oxo-MeBmt]¹-[val ciclosporin (PCS833), [3'-desoxy-3'-oxo-MeBmt]¹-[Nva]²-Ciclosporin, Cyclo-[Pec-MeVal-Val-MeAsp(β-P-t-Bu)-MeIle-MeIle-GlyMeVal-Tyr(Me)-L-Lact], and cyclo-[Pec-MeVal-Val-MeAso-MeIle-MeIle-Gly-MeVal-Tyr(Me)-D-Lact] and an antiepileptic drug selected from phenytoin (5,5-diphenyl-2,4-imidazolidinedione), carbamazepine, lamotrigine, gabapentin, oxcarbazepin, valproic acid, and topiramate for simultaneous, separate or sequential use in the prevention, delay of progression or treatment of diseases, in particular epilepsy, especially epilepsy which is resistant to antiepileptic drugs; the use of such combination for the preparation of a medicament for such prevention, delay of progression or treatment; and to a method of prevention, delay of progression or treatment of epilepsy.

Resistance to antiepileptic drugs is a major problem in the treatment of epilepsy. The mechanisms underlying the development of chronic or pharmacoresistant epilepsy are far from being understood. As known from the prior art, most antiepileptic drugs enter the brain by diffusion and not by active transport mechanisms. Surprisingly, it was found that the administration of a combination disclosed herein result in an increased local concentration of the antiepileptic drug in the brain without enhancing the side-effects of such drug by the same factor as such local concentration or, preferably, without enhancing the side-effects of such drug at all. Such finding qualifies the combinations disclosed herein to be more suitable to treat epilepsy which is resistant to antiepileptic drugs than the corresponding antiepileptic drugs alone.

The present invention relates to a combination, such as a combined preparation or pharmaceutical composition, respectively, which comprises a P-gp inhibitor selected from [3'-desoxy-3'-oxo-MeBmt]¹-Ciclosporin[3'-desoxy-3'-oxo-MeBmt]¹-[val ciclosporin (PCS833), [3'-desoxy-3'-oxo-MeBmt]¹-[Nva]²-Ciclosporin, Cyclo-[Pec-MeVal-Val-MeAsp(β-P-t-Bu)-MeIle-MeIle-GlyMeVal-Tyr(Me)-L-Lact[, and cyclo-[Pec-MeVal-Val-MeAso-MeIle-MeIle-Gly-MeVal-Tyr(Me)-D-Lact] and an antiepileptic drug selected from phenytoin, carbamazepine, lamotrigine, gabapentin, oxcarbazepin, valproic acid, and topiramate, in which the active ingredients are present in each case in free form or in the form of a pharmaceutically acceptable salt and optionally at least one pharmaceutically acceptable carrier; for simultaneous, separate or sequential use, particularly, in the prevention, delay of progression or treatment of diseases, in particular epilepsy, especially epilepsy which is resistant to antiepileptic drugs. Such a combination is preferably a combined preparation or a pharmaceutical composition.

By the term "a combined preparation or pharmaceutical composition for simultaneous, separate or sequential use", there is meant especially a "kit of parts" in the sense that the components P-gp inhibitor and an antiepileptic drug selected from phenytoin, carbamazepine, lamotrigine, gabapentin, oxcarbazepin, valproic acid, and topiramate can be dosed independently or by use of different fixed combinations with distinguished amounts of the components, i.e. at different time points or simultaneously. The parts of the kit of parts can then e.g. be administered simultaneously or chronologically staggered, that is at different time points and with equal or different time intervals for any part of the kit of parts. Preferably, the time intervals are chosen such that the effect on the treated disease or condition in the combined use of the parts is larger than the effect which would be obtained by use of only any one of the components.

The term "prevention" means prophylactic administration of the combination to healthy patients to prevent the outbreak of the diseases and conditions mentioned herein. Moreover, the term "prevention" means prophylactic administration of such combination to patients being in a pre-stage of the disease to be treated. The term "delay of progression" used herein means administration of the combination to patients being in a pre-stage of the disease to be treated in which patients a pre-form of the corresponding disease is diagnosed.

The term "pharmacoresistant" or "pharmacoresistance" as used herein in conjunction with epilepsy relates to epilepsy which is refractory to the treatment with two or, preferably, three antiepileptic drugs applied in a dosage and during a term which constitute about the standard regimen for said drugs.

The term "P-gp inhibitor" as used herein relates to compounds which inhibit the activity of the P-glycoprotein. The term includes, [3'-desoxy-3'-oxo-MeBmt]¹-Ciclosporin, [3'-desoxy-3'-oxo-MeBmt]¹-[Val]²-Ciclosporin and [3'-desoxy-3'-oxo-MeBmt]¹-[Nva]²-Ciclosporin disclosed in EP 0 296 122 in Example H as cyclosporins 1.37, 1.38 and 1.39, respectively, as well as Cyclo-[Pec-MeVal-Val-MeAsp(β-P-t-Bu)-MeIle-MeIle-Gly-MeVal-Tyr(Me)-L-Lact] and Cyclo-[Pec-MeVal-Val-MeAsp-MeIle-MeIle-Gly-MeVal-Tyr(Me)-D-Lact], disclosed in EP 0 360 760 as Examples 52 and 1 (first compound), respectively. With regard to all aspects of the present invention, preferably [3'-desoxy-3'-oxo-MeBmt]¹-[Val]²-Ciclosporin A, also known as valspodar, hereinafter referred to as PSC833, known from EP 0 296 122 (Example H) is used as the P-gp inhibitor. PSC833 can be administered in the form of the galenical composition disclosed in WO 93/20833.

5,5-Diphenyl-2,4-imidazolidinedione, also known as phenytoin, can be prepared as disclosed in US 2,409,754 and administered, e.g., in the form as it is marketed, e.g. under the trademark ZENTROPIL™, LEHYDAN™, PHENHYDAN™ or DIFHYDAN™. It can also be used in the form of its sodium salt.

Carbamazepine can be prepared as described in US 2,948,718. It can be administered, e.g., in the form as it is marketed, e.g. under the trademarks CALEPSIN™ or TEGRETOL™.

Lamotrigine can be prepared as described in US 4,602,017. It can be administered, e.g., in the form as it is marketed, e.g. under the trademarks LAMICTAL™ or LAMICTAL CD™.

Gabapentin can be prepared as described in US 4,024,175. It can be administered, e.g., in the form as disclosed in US 4,087,544 or in the form as marketed, e.g. under the trademark NEURONTIN™.

Topiramate can be prepared as described in US 4,513,006. It can be administered, e.g., in the form as it is marketed, e.g. under the trademarks TOPOMAX™ or TOPOMAX SPRINKLE™.

Valproic acid can be administered, e.g., in the form as it is marketed, e.g. under the trademark CONVULEX™. Furthermore, it can be administered in the form of its sodium salt, e.g. as it is marketed under the trademark VALPROAT AZU™.

Oxcarbazepin can be administered, e.g., in the form as it is marketed, e.g. under the trademark TRILEPTAL™.

Further diseases that can be treated by one or more of the combinations disclosed herein are especially anxiety, pain, psychosis, migraine and depression.

The active ingredients or a pharmaceutically acceptable salt thereof may also be used in form of a hydrate or include other solvents used for crystallization.

The structure of the active agents identified by code nos., generic or trade names may be taken from the actual edition of the standard compendium "The Merck Index" or from databases, e.g. Patents International (e.g. IMS World Publications).

For the treatment of epilepsy, especially epilepsy which is resistant to antiepileptic drugs, the P-gp inhibitor is preferably selected from [3'-desoxy-3'-oxo-MeBmt]¹-Ciclosporin, [3'-desoxy-3'-oxo-MeBmt]¹-[Val]²-Ciclosporin, [3'-desoxy-3'-oxo-MeBmt]¹-[Nva]²-Ciclosporin, Cyclo-[Pec-MeVal-Val-MeAsp(β-P-t-Bu)-MeIle-MeIle-Gly-MeVal-Tyr(Me)-L-Lact] and Cyclo-[Pec-MeVal-Val-MeAsp-MeIle-MeIle-Gly-MeVal-Tyr(Me)-D-Lact], more preferably the P-gp inhibitor is PSC833.

It can be shown by established test models and especially the test model described herein that the combination of a P-gp inhibitor, and an antiepileptic drug selected from carbamazepine, lamotrigine, gabapentin, oxcarbazepin, valproic acid, topiramate and, especially, phenytoin (5,5-diphenyl-2,4-imidazolidinedione), or in each case a pharmaceutically acceptable salt thereof, results in a more effective prevention or preferably treatment of epilepsy, especially epilepsy which is resistant to antiepileptic drugs, e.g., phenytoin in the absence of a P-gp inhibitor. The pharmacological activity may, for example, be demonstrated following essentially the *in-vivo* test procedure in rats or in a clinical study as described hereinafter.

### In vivo microdialysis in adult female Wistar rats

The combination of phenytoin (Aldrich, Steinheim, Germany) and a P-gp inhibitor, e.g. PSC833) is given via the microdialysis probe in the right frontal cortex, while a probe in the left cortex served as a vehicle control site. Perfusion with the P-gp inhibitor started 15 to 60 minutes prior to i.p. administration of 50 mg/kg phenytoin.

Animals: Adult female Wistar rats (Harlan-Winkelmann, Germany) kept under controlled environmental conditions are used in the study.

Implantation of guide cannulae: Guide cannulae (CMA/12 polyurethane, Camegie Medicine, Sweden) are implanted into the left and right frontal (motor) cortex under anesthesia. The tips of the guide are positioned at rostral + 3.2, lateral + 3.2 or 3.2 and ventral 2.0 mm to bregma, coordinates according to Paxinos and Watson, The rat brain in stereotaxic coordinates, Sydney, Academic Press, 1986.

Microdialysis procedure: Microdialysis experiments are performed following a recovery period of at least 3 days after surgery. The microdialysis probe is lowered through the guide cannula to a depth of 5.0 mm according to bregma. 14 to 16 h after insertion, perfusion of the probe is started using Ringer solution (in mM 147 Na⁺, 2.3 Ca ²⁺, 4.0 K⁺ and 155.6 Cl⁻, pH 6.0). Two dialysate samples are collected over a time period of 1 h before rats are injected with phenytoin (50 mg/kg i.p.). Following drug administration, further 4 samples are collected over the next 2 h. Local application of the P-gp inhibitor, e.g. 2 mM PSC833, via the right microdialysis probe is started 15 min prior to the phenytoin injection. The left microdialysis probe is perfused with the respective drug vehicle, e.g. Ringer solution with 15 % cremophor EL and 3 % ethanol in the case of PSC833.

High pressure liquid chromatography (HPLC): Phenytoin concentrations in dialysate and plasma samples are determined by HPLC with UV detection.

Results: In the absence of a P-gp inhibitor, extracellular levels of phenytoin in the left and right cerebral cortex increase rapidly, reaching maximum levels of about 200 to 1150 ng/ml within 60 to 90 minutes following systemic injection of phenytoin in individual rats. After maximum levels have been reached, ECF concentrations of phenytoin decrease with an average half-life of about 4 h. For example, PSC833 increases ECF levels of phenytoin in nearly all rats, the maximum increase being 70 ± 20 % compared to untreated site. When the ECF plasma ratio of the PSC833 treated site is compared to ECF plasma ratios of vehicle treated controls, ECF levels of phenytoin are increased by about 150 % above control.

By the study in rats described herein before it is demonstrated that the concentration of phenytoin in the extracellular fluid (ECF) of the cerebral cortex can be enhanced by co-application of a P-gp inhibitor, especially PSC833.

A further advantage of the present combination is the fact that the antiepileptic drug selected from phenytoin, carbamazepine, lamotrigine, gabapentin, oxcarbazepin , and topiramate can be applied, at least in some patients, in a lower dosage in the prevention, delay of progression or treatment of epilepsy which is not resistant to antiepileptic drugs and also in cases where generally higher doses of the antiepileptic drug would be needed in order to effect alleviation from epilepsy, e.g., due to the first onset of resistance to such antiepileptic drug. A lower dosage of the antiepileptic drug results normally in less side-effects.

Furthermore, the present invention relates to a combined preparation which comprises a P-gp inhibitor and an antiepileptic drug in which the active ingredients are present in each case in free form or in the form of a pharmaceutically acceptable salt and optionally at least one pharmaceutically acceptable carrier, as a combined preparation for simultaneous, separate or sequential use.

It is one objective of this invention to provide a pharmaceutical composition comprising an amount, which is jointly therapeutically effective in epilepsy which is resistant to antiepileptic drugs, of (i) a P-gp inhibitor and (ii) an antiepileptic drug or a pharmaceutically acceptable salt thereof and at least one pharmaceutically acceptable carrier. In this composition, the components (i) and (ii) can be administered together, one after the other or separately in one combined unit dosage form or in two separate unit dosage forms. The unit dosage form may also be a fixed combination.

The pharmaceutical compositions according to the invention can be prepared in a manner known per se and are those suitable for enteral, such as oral or rectal, and parenteral administration to mammals (warm-blooded animals), including man, comprising a therapeutically effective amount of the pharmacologically active compound, alone or in combination with one or more pharmaceutically acceptable carriers, especially suitable for enteral or parenteral application.

The novel pharmaceutical preparations contain, for example, from about 10 % to about 100 %, preferably 80%, preferably from about 20 % to about 60 %, of the active ingredient. Pharmaceutical preparations for the combination therapy that may be used for enteral or parenteral administration are, for example, those in unit dose forms, such as sugar-coated tablets, tablets, capsules or suppositories, and furthermore ampoules. If not indicated otherwise, these are prepared in a manner known per se, for example by means of conventional mixing, granulating, sugar-coating, dissolving or lyophilizing processes. Thus, pharmaceutical preparations for oral use can be obtained by combining the active ingredient with solid carriers, if desired granulating a mixture obtained, and processing the mixture or granules, if desired or necessary, after addition of suitable excipients to give tablets or sugar-coated tablet cores.

It will be appreciated that the unit content of active ingredient or ingredients contained in an individual dose of each dosage form need not in itself constitute an effective amount since the necessary effective amount can be reached by administration of a plurality of dosage units.

In particular, a therapeutically effective amount of each of the components of the combination of the present invention may be administered simultaneously or sequentially and in any order, and the components may be administered separately or as a fixed combination. The individual components of the combination can be administered separately at different times during the course of therapy or concurrently in divided or single combination forms. Furthermore, the term administering also encompasses the use of prodrugs of any of the drugs that convert in vivo to the selective drugs. The instant invention is therefore to be understood as embracing all such regimes of simultaneous or altemating treatment and the term "administering" is to be interpreted accordingly.

The preferred route of administration of the dosage forms of the present invention is enterally or, preferably, orally. Because of their ease of administration, tablets and capsules represent the most advantageous oral dosage unit form in which case solid pharmaceutical carriers are obviously employed.

The effective dosage of each of the active ingredients employed in the combination therapy may vary depending on the particular pharmaceutical composition employed, the mode of administration, or the severity of the condition being treated. A physician, clinician or veterinarian of ordinary skill can readily determine and prescribe the effective amount of the drug required to prevent, counter or arrest the progress of the condition.

A further aspect of the present invention is the use of a pharmaceutical composition comprising a P-gp inhibitor and antiepileptic drug selected from phenytoin, carbamazepine, lamotrigine, gabapentin, oxcarbazepin, valproic acid, and topiramate in free form or in form of a pharmaceutically acceptable salt thereof for the preparation of a medicament for the prevention, delay of progression or treatment of epilepsy, especially epilepsy which is resistant to antiepileptic drugs.

In accordance with the present invention there is further provided a method of prevention, delay of progression or treatment of and a pharmaceutical composition for the prevention, delay of progression or treatment of epilepsy, especially epilepsy which is resistant to antiepileptic drugs. The treatment involves administering to a patient in need of such treatment a pharmaceutical composition comprising a pharmaceutical carrier and a therapeutically effective amount of each compound in the combination of the present invention.

In one embodiment of the invention a combination as disclosed herein is administered locally to the brain of a mammal, especially a human, suffering from epilepsy or another disease mentioned herein. Such a local administration can, e.g., be accomplished by means of a small pump placed under the skin of the mammal, which pump, e.g. continuously, provides such combination to a particular region of the brain. Hence, the present invention pertains also to the use of a combination as disclosed herein for the preparation of a medicament wherein the medicament is adapted for local administration to a particular region of the brain of a mammal.

The invention relates in particular to a commercial package comprising jointly therapeutically effective amounts of a P-glycoprotein (P-gp) inhibitor and antiepileptic drug, in free or pharmaceutically acceptable salt form in each case, together with instructions for use thereof in the treatment of epilepsy, especially epilepsy which is resistant to antiepileptic drugs, anxiety, pain, psychosis, migraine or depression.

PSC833 is preferably administered to a human in a dosage in the range of about 50 to 1000, more preferably 100 to 500 mg/day.

Phenytoin is preferably administered orally to a human in a dosage in the range of about 50 to 400, more preferably 100 to 300 mg/day.

Carbamazepine is preferably administered orally to a human in a dosage in the range of about 200 to 1600, more preferably 200 to 600 mg/day.

Lamotrigine is preferably administered orally to a human in a dosage in the range of about 10 to 500, more preferably 25 to 250 mg/day.

Gabapentin, is preferably administered orally to a human in a dosage in the range of about 300 to 3000, more preferably 900 to 2400 mg/day.

Oxcarbazepin is preferably administered orally to a human in a dosage in the range of about 150 to 3000 mg/day.

Valproic acid is preferably administered orally to a human in a dosage in the range of about 150 to 2500 mg/day.

Topiramate is preferably administered orally to a human in a dosage in the range of about 250 to 1000, more preferably 50 to 400 mg/day.

## Claims

1. Combination which comprises a P-glycoprotein (P-gp) inhibitor selected from [3'-desoxy-3'-oxo-MeBmt]¹-Ciclosporin, [3'-desoxy-3'-oxo-MeBmt]¹-[Val]²-Ciclosporin (PCS833), [3'-desoxy-3'-oxo-MeBmt]¹-[Nva]²-Ciclosporin, Cyclo-[Pec-MeVal-Val-MeAsp(β-P-t-Bu)-MeIle-MeIle-Gly-MeVal-Tyr(Me)-L-Lact], and Cyclo-[Pec-MeVal-Val-MeAsp-MeIle-MeIle-Gly-MeVal-Tyr(Me)-D-Lact] and an antiepileptic drug selected from phenytoin, carbamazepine, lamotrigine, gabapentin, oxcarbazepin, valproic acid, and topiramate, in which the active ingredients are present in each case in free form or in the form of a pharmaceutically acceptable salt and optionally at least one pharmaceutically acceptable carrier; for simultaneous, separate or sequential use.

2. Combination according to claim 1 which is a combined preparation or a pharmaceutical composition.

3. Combination according to claim 1 or 2, **characterized in that** the P-gp inhibitor is PSC833.

4. Combination according to claim 3 **characterized in that** PSC833 is administered to a human in a dosage in the range of about 50 to 1000 mg/day.

5. Combination according to claim 4 **characterized in that** PSC833 is administered to a human in a dosage in the range of about 100 to 500 mg/day.

6. Combination according to any one of claims 1 to 5 for simultaneous, separate or sequential use in the prevention, delay of progression or treatment of epilepsy, anxiety, pain, psychosis, migraine or depression.

7. A pharmaceutical composition comprising a combination according to any one of claims 1 to 6 in a quantity which is therapeutically effective against epilepsy and at least one pharmaceutically acceptable carrier.

8. A pharmaceutical composition according to claim 8 wherein the epilepsy is an epilepsy resistant to antiepileptic drugs.

9. Use of a combination according to any one of claims 1 to 6 for the preparation of a medicament for the prevention, delay of progression or treatment of anxiety, pain, psychosis, migraine or depression.

10. Use of a combination according to any one of claims 1 to 6 for the preparation of a medicament for the prevention, delay of progression or treatment of epilepsy.

11. Use according to claim 10, **characterized in that** the epilepsy is pharmacoresistant.

12. Use according to any one of claims 9 to 11, wherein the medicament is adapted for local administration to a particular region of the brain of a mammal.

13. A commercial package comprising as active agent a P-glycoprotein (P-gp) inhibitor selected from [3'-desoxy-3'-oxo-MeBmt]¹-Ciclosporin, [3'-desoxy-3'-oxo-MeBmt]¹-[Val]²-Ciclosporin (PCS833), [3'-desoxy-3'-oxo-MeBmt]¹-[Nva]²-Ciclosporin, Cyclo-[Pec-MeVal-Val-MeAsp(β-P-t-Bu)-MeIle-MeIle-Gly-MeVal-Tyr(Me)-L-Lact], and Cyclo-[Pec-MeVal-Val-MeAsp-MeIle-MeIle-Gly-MeVal-Tyr(Me)-D-Lact] and an antiepileptic drug selected from phenytoin, carbamazepine, lamotrigine, gabapentin, oxcarbazepin, valproic acid, and topiramate, together with instructions for simultaneous, separate or sequential use thereof in the prevention, delay of progression or treatment of epilepsy, anxiety, pain, psychosis, migraine or depression.

## Patentansprüche

1. Kombination, die einen P-Glykoprotein (P-Gp)-Inhibitor, der ausgewählt ist aus [3'-Desoxy-3'-oxo-MeBmt]¹-Ciclosporin, [3'-Desoxy-3'-oxo-MeBmt]¹-[Val]²-Ciclosporin (PCS833), [3'-Desoxy-3'-oxo-MeBmt]¹-[Nva]²-Ciclosporin, Cyclo-[Pec-MeVal-Val-MeAsp(β-P-t-Bu)-Melle-Melle-Gly-MeVal-Tyr(Me)-L-Lact] und Cyclo-[Pec-MeVal-Val-MeAsp-Melle-Melle-Gly-MeVal-Tyr(Me)-D-Lact], und ein antiepileptisches Arzneimittel, das ausgewählt ist aus Phenytoin, Carbamazepin, Lamotrigin, Gabapentin, Oxcarbazepin, Valproinsäure und Topiramat, in der die wirksamen Inhaltsstoffe jeweils in freier Form oder in der Form eines pharmazeutisch annehmbaren Salzes vorliegen, und gegebenenfalls mindestens einen pharmazeutisch annehmbaren Träger umfasst, zur gleichzeitigen, getrennten oder sequenziellen Verwendung.

2. Kombination gemäß Anspruch 1, die eine kombinierte Zubereitung oder eine pharmazeutische Zusammensetzung ist.

3. Kombination gemäß Anspruch 1 oder 2, **dadurch gekennzeichnet, dass** der P-Gp-Inhibitor für PSC833 steht.

4. Kombination gemäß Anspruch 3, **dadurch gekennzeichnet, dass** PSC833 einem Menschen in einer Dosierung in dem Bereich von etwa 50 bis 1000 mg/Tag verabreicht wird.

5. Kombination gemäß Anspruch 4, **dadurch gekennzeichnet, dass** PSC833 einem Menschen in einer Dosierung in dem Bereich von etwa 100 bis 500 mg/Tag verabreicht wird.

6. Kombination gemäß einem der Ansprüche 1 bis 5 zur gleichzeitigen, getrennten oder sequenziellen Verwendung bei der Vorbeugung, Verzögerung des Fortschreitens oder Behandlung von Epilepsie, Angst, Schmerz, einer Psychose, Migräne oder Depression.

7. Pharmazeutische Zusammensetzung, umfassend eine Kombination gemäß einem der Ansprüche 1 bis 6 in einer Menge, die therapeutisch wirksam ist gegen Epilepsie und mindestens einen pharmazeutisch annehmbaren Träger.

8. Pharmazeutische Zusammensetzung gemäß Anspruch 8, wobei die Epilepsie eine Epilepsie ist, die resistent ist gegen antiepileptische Arzneimittel.

9. Verwendung von einer Kombination gemäß einem der Ansprüche 1 bis 6 zur Herstellung eines Arzneimittels für die Vorbeugung, Verzögerung des Fortschreitens oder Behandlung von Angst, Schmerz, einer Psychose, Migräne oder Depression.

10. Verwendung von einer Kombination gemäß einem der Ansprüche 1 bis 6 zur Herstellung eines Medikaments für die Vorbeugung, Verzögerung des Fortschreitens oder Behandlung von Epilepsie.

11. Verwendung gemäß Anspruch 10, **dadurch gekennzeichnet, dass** die Epilepsie pharmakoresistent ist.

12. Verwendung gemäß einem der Ansprüche 9 bis 11, wobei das Arzneimittel eingerichtet ist zur lokalen Verabreichung an eine besondere Region des Hirns von einem Säuger.

13. Kommerzielle Verpackung, umfassend als wirksames Mittel einen P-Glykoprotein (P-Gp)-Inhibitor, der ausgewählt ist aus [3'-Desoxy-3'-oxo-MeBmt]¹-Ciclosporin, [3'-Desoxy-3'-oxo-MeBmt]¹-[Val]²-Ciclosporin (PCS833), [3'-Desoxy-3'-oxo-MeBmt]¹-[Nva]²-Ciclosporin, Cyclo-[Pec-MeVal-Val-MeAsp(β-P-t-Bu)-Melle-Melle-Gly-MeVal-Tyr(Me)-L-Lact] und Cyclo-[Pec-MeVal-Val-MeAsp-Melle-Melle-Gly-MeVal-Tyr(Me)-D-Lact], und ein antiepileptisches Arzneimittel, das ausgewählt ist aus Phenytoin, Carbamazepin, Lamotrigin, Gabapentin, Oxcarbazepin, Valproinsäure und Topiramat, zusammen mit Anleitungen zur simultanen, getrennten oder sequenziellen Verwendung davon bei der Vorbeugung, Verzögerung des Fortschreitens oder zur Behandlung von Epilepsie, Angst, Schmerz, einer Psychose, Migräne oder Depression.

## Revendications

1. Combinaison comprenant un inhibiteur de la P-glycoprotéine (P-gp) sélectionné à partir de [3'-desoxy-3'-oxo-MeBmt]¹-Ciclosporine, [3'-desoxy-3'-oxo-MeBmt]¹-[Val]²-Ciclosporine (PCS833), [3'-desoxy-3'-oxo-MeBmt]¹-[Nva]²-Ciclosporine, Cyclo-[Pec-MeVal-Val-MeAsp(β-P-t-Bu)-MeIle-MeIle-Gly-MeVal-Tyr(Me)-L-Lact] et Cyclo-[Pec-MeVal-Val-MeAsp-MeIle-MeIle-Gly-MeVal-Tyr(Me)-D-Lact] et un produit anti-épileptique sélectionné à partir de phénytoïne, carbamazépine, lamotrigine, gabapentine, oxcarbazépine, acide valproïque et topiramate, dans laquelle les ingrédients actifs sont présents, dans chacun des cas, sous forme libre ou sous forme d'un sel pharmaceutiquement acceptable et comprenant, éventuellement, au moins un véhicule pharmaceutiquement acceptable ; pour une utilisation simultanée, séparée ou séquentielle.

2. Combinaison selon la revendication 1 qui est une préparation combinée ou une composition pharmaceutique.

3. Combinaison selon la revendication 1 ou 2 **caractérisée en ce que** l'inhibiteur de la P-gp est PSC833.

4. Combinaison selon la revendication 3 **caractérisée en ce que** PSC833 est administré à un être humain selon un dosage compris dans la gamme allant d'environ 50 à 1 000 mg/jour.

5. Combinaison selon la revendication 4 **caractérisée en ce que** PSC833 est administré à un être humain selon un dosage compris dans la gamme allant d'environ 100 à 500 mg/jour.

6. Combinaison selon l'une des revendications 1 à 5 destinée à une utilisation simultanée, séparée ou séquentielle dans la prévention, le ralentissement de la progression ou le traitement de l'épilepsie, de l'anxiété, de la douleur, de la psychose, de la migraine ou de la dépression.

7. Composition pharmaceutique comprenant une combinaison selon l'une quelconque des revendications 1 à 6 selon une quantité thérapeutiquement efficace à l'encontre de l'épilepsie ainsi qu'au moins un véhicule pharmaceutiquement acceptable.

8. Composition pharmaceutique selon la revendication 8 **caractérisée en ce que** l'épilepsie est une épilepsie résistante aux produits anti-épileptiques.

9. Utilisation d'une combinaison selon l'une quelconque des revendications 1 à 6 pour la préparation d'un médicament pour la prévention, le ralentissement de la progression ou le traitement de l'anxiété, de la douleur, de la psychose, de la migraine ou de la dépression.

10. Utilisation d'une combinaison selon l'une quelconque des revendications 1 à 6 pour la préparation d'un médicament pour la prévention, le ralentissement de la progression ou le traitement de l'épilepsie.

11. Utilisation selon la revendication 10 **caractérisée en ce que** l'épilepsie est pharmacorésistante.

12. Utilisation selon l'une quelconque des revendications 9 à 11, **caractérisée en ce que** le médicament est adapté de façon à être administré localement au niveau d'une région déterminée du cerveau d'un mammifère.

13. Conditionnement commercial comprenant, en tant qu'ingrédient actif, un inhibiteur de la P-glycoprotéine (P-gp) sélectionné à partir de [3'-desoxy-3'-oxo-MeBmt]¹-Ciclosporine, [3'-desoxy-3'-oxo-MeBmt]¹-[Val]²-Ciclosporine (PCS833), [3'-desoxy-3'-oxo-MeBmt]¹-[Nva]²-Ciclosporine, Cyclo-[Pec-MeVal-Val-MeAsp(β-P-t-Bu)-MeIle-MeIle-Gly-MeVal-Tyr(Me)-L-Lact] et Cyclo-[Pec-MeVal-Val-MeAsp-MeIle-MeIle-Gly-MeVal-Tyr(Me)-D-Lact] et un produit anti-épileptique sélectionné à partir de phénytoïne, carbamazépine, lamotrigine, gabapentine, oxcarbazépine, acide valproïque et topiramate de pair avec des instructions pour une utilisation simultanée, séparée ou séquentielle pour la prévention, le ralentissement de la progression ou le traitement de l'épilepsie, de l'anxiété, de la douleur, de la psychose, de la migraine ou de la dépression.
